Europäisches Patentamt

European Patent Office       (11) Numéro de publication : **0 149 502**

Office européen des brevets                              **B1**

(19)

(12)                   **FASCICULE DE BREVET EUROPÉEN**

(45) Date de publication du fascicule du brevet :       (51) Int. Cl.⁴ : **A 61 B   6/14**
26.04.89

(21) Numéro de dépôt : 85200026.4

(22) Date de dépôt : 15.01.85

(54) Appareillage dentaire de détection de rayons X comportant un outil dentaire de prélèvement en bouche d'images photoniques.

(30) Priorité : 17.01.84 FR 8400636

(43) Date de publication de la demande :
24.07.85 Bulletin 85/30

(45) Mention de la délivrance du brevet :
26.04.89 Bulletin 89/17

(84) Etats contractants désignés :
DE FR GB IT

(56) Documents cités :
US--A-- 3 051 166
US--A-- 3 556 085
US--A-- 3 622 785
US--A-- 4 015 592
US--A-- 4 160 997
US--A-- 4 259 583
US--A-- 4 323 779

(73) Titulaire : **Laboratoires d'Electronique et de Physique
Appliquée L.E.P.
3, Avenue Descartes
F-94450 Limeil-Brévannes (FR)
FR
N.V. Philips' Gloeilampenfabrieken
Groenewoudseweg 1
NL-5621 BA Eindhoven (NL)
DE GB IT**

(72) Inventeur : **Martin, Guy
Société Civile S.P.I.D. 209, rue de l'Université
F-75007 Paris (FR)**
Inventeur : **Richard, Jean-Claude
Société Civile S.P.I.D. 209, rue de l'Université
F-75007 Paris (FR)**
Inventeur : **Bouchier, Guy
Société Civile S.P.I.D. 209, rue de l'Université
F-75007 Paris (FR)**

(74) Mandataire : **Landousy, Christian et al
Société Civile S.P.I.D. 209, Rue de l'Université
F-75007 Paris (FR)**

## Description

L'invention concerne un appareillage dentaire de détection de rayons X permettant d'acquérir en temps réel des images d'un champ opératoire couvert par un faisceau de rayons X issu d'une source de rayons X, comprenant un scintillateur placé sur une extrémité d'un conduit d'images à fibres optiques dont l'autre extrémité est reliée à un amplificateur de luminance couplé à un dispositif de prise de vue électronique, le scintillateur étant placé en bouche.

En dentisterie, il est connu de réaliser des images radioscopiques en temps réel en utilisant une source de rayons X extra-orale et un détecteur de rayons X intra-oral. Ceci est décrit dans le brevet des Etats-Unis n° 3 622 785. Il y est décrit un conduit d'images à fibres optiques portant un scintillateur sur l'extrémité en bouche. Ce conduit d'images à fibres optiques est courbé pour atteindre la zone à examiner. l'autre extrémité du conduit d'images à fibres optiques est relié à un intensificateur d'images, lui-même relié à un dispositif de prise de vue constitué d'un tube vidicon. les signaux électriques ainsi délivrés sont exploités pour former une image sur un téléviseur ou enregistrés à l'aide d'un magnétoscope. Or ce dispositif présente des inconvénients, car il résoud mal le problème de la maniabilité et de la compacité de l'outil dentaire que le praticien doit utiliser. Un outil dentaire doit avoir des dimensions les plus faibles possibles être léger et avoir une forme s'adaptant le mieux possible au champ opératoire c'est-à-dire à la bouche du patient.

Le dispositif connu de l'art antérieur ne répond pas à ces critères. les tubes vidicons même les plus récents sont des tubes qui présentent une longueur élevées d'où un encombrement important. D'autre parts pour fonctionner ils ont besoin d'une haute tension et de signaux de balayage. lorsque le tube vidicon est placé contre le tube intensificateur d'images la proximité des deux hautes tensions des deux tubes, conduit à une interaction mutuelle ce qui se traduit par des perturbations de l'image, ce qui peut présenter des conséquences graves compte tenu du domaine d'application. Pour diminuer ces effets on peut espacer les deux tubes mais pour conserver l'image il est alors nécessaire d'insérer un objectif optique entre les deux tubes. Ceci a pour conséquence d'allonger et d'alourdir l'outil dentaire. Comme le praticien doit effectuer un travail de précision, en manipulant l'outil dentaire très près de la bouche tout outil d'assez grande longueur et quelque peu alourdi, va présenter un porte-à-faux qui va le rendre très difficilement manipulable par le praticien.

D'autre part, si l'extrémité en bouche d'un conduit d'images est coudées elle va présenter des dimensions non négligeables. Le scintillateur porté par l'extrémité du conduit d'images doit avoir une surface suffisante pour observer l'ensemble d'une dent. Il s'ensuit que le conduit d'images à fibres optiques va présenter un diamètre tel que la courbure qui va être acceptable par le conduit d'images va être effectuée sur une assez grande longueur. Les contraintes de courbure qui en découlent vont avoir des conséquences sur la qualité de l'image en particulier sur le contraste. En effet un conduit de lumière courbé va présenter une résolution d'image diminuée à cause de phénomènes d'interaction optique entre les fibres optiques élémentaires. Selon l'art antérieur la longueur totale de l'outil dentaire formé d'un conduit d'images coudés d'un tube intensificateur d'images et d'un tube vidicon, et éventuellement d'une optique intermédiaire, va ainsi devenir prohibitive pour constituer un outil réellement maniable par le praticien.

En conséquence un but de l'invention est de déterminer un outil de faibles dimensions, qui soit aisément manipulable par le praticien, sans porte-à-faux, et qui n'encombre pas le champ opératoire, de telle sorte que le praticien puisse le laisser en bouche tout en continuant ses interventions afin de suivre celles-ci au gré de la nécessité.

Un autre but de l'invention est d'assurer un maximum de contraste à l'image, en diminuant le moins possible la résolution intrinsèque de la fibre optique.

L'invention concerne ainsi un appareillage dentaire du genre décrit dans le préambule caractérisé en ce que la partie en bouche du conduit d'image à fibres optiques présente une face biseautée formée par une section non-droite du conduit d'images de sorte que la direction de tenue en bouche du conduit d'image n'est pas colinéaire avec la direction du faisceau de rayons X, et en ce que le dispositif de prise de vue est un dispositif à transfert de charges bidimensionnel.

Selon une variante, le scintillateur est déposé sur la face biseautée du conduit d'images à fibres optiques.

Selon une autre variante la face biseautée est accolée à une face d'un élément à renvoi d'angle à fibres optiques dont l'autre face porte le scintillateur.

Le conduit d'images à fibres optiques peut être constitué d'une partie rigide qui porte le scintillateur et d'une partie souple formée d'un conduit d'images souple à fibres optiques. L'outil dentaire possède une très grande maniabilité. L'ouverture buccale assure une bonne accessibilité pour une intervention dentaire dans la mesure où les matériels que l'on désire y introduire sont de faibles dimensions et très maniables. Pour permettre cette grande maniabilité, l'outil dentaire en disposant d'une bonne préhension par la main du praticien, comprend un conduit d'images rigide à fibres optiques, transparent optiquement, suffisamment long pour atteindre l'ensemble du champ opératoire, et pouvant détecter et transformer les rayons X en photons, puis véhiculer les images photoniques détectées vers l'extérieur de la bouche du patient. Ce conduit d'images rigide à fibres optiques est muni d'un scintillateur à l'extrémité en bouche. Les dimensions de celui-ci sont proportionnées aux dimensions de la zone à étudier. La partie de l'outil dentaire qui sert à intensifier l'image et à la restituer sous la forme d'un signal électrique, tient dans la paume de la main.

Le conduit d'images présente une face biseautée sur la partie située en bouche. Cette face biseautée peut recevoir le scintillateur soit directement soit par l'intermédiaire d'un élément à renvoi d'angle. Le conduit d'images rigide à fibres optiques peut être muni dans la zone en bouche d'un raccord à fibres optiques à concentration qui permet de réduire les dimensions des images photoniques de la zone étudiée pour leur acheminement vers l'extérieur du champ opératoire. Egalement pour que l'outil dentaire soit léger, de faibles dimensions, et assure une facilité maximale d'accès aux différentes zones à atteindre dans le champ opératoire, on peut, dans la partie en bouche, munir le conduit d'images rigide à fibres optiques d'un raccord à renvoi d'angle à concentration.

Le conduit d'images rigide à fibres optiques est pourvu d'une gaine externe empêchant la pénétration de tout flux lumineux parasite. La partie de la gaine externe qui se trouve irradiée par le faisceau de rayons X peut être constituée d'un matériau absorbant les rayons X afin que les rayons X présents au-delà du scintillateur soient absorbés pour ne pas irradier inutilement les tissus situés au-delà.

Le praticien a la possibilité de prendre en main l'outil dentaire et d'opérer la prise d'images photoniques. Egalement lorsqu'une observation globale du champ opératoire s'impose ou lorsque le praticien désire effectuer simultanément d'autres interventions en bouche, l'outil dentaire peut être monté sur un arceau porté par la source de rayons X. L'outil dentaire et la source de rayons X se trouvent ainsi positionnés correctement et rigidement l'un par rapport à l'autre. La source est montée sur une potence mobile qui permet au praticien de déplacer solidairement l'outil dentaire et le faisceau de rayons X dans la zone à étudier et d'observer et/ou de stocker dans un dispositif de stockage, le panoramique ainsi prélevé. On obtient ainsi un appareillage dentaire qui comprend la source de rayons X et l'outil dentaire qui sont positionnés rigidement l'un par rapport à l'autre à l'aide d'un arceau afin de pouvoir les déplacer solidairement de sorte que leurs axes respectifs de symétrie concourent juste en arrière du scintillateur.

Pour permettre le contrôle en temps réel, l'outil dentaire travaille avec une dose très faible de rayons X irradiant le volume de champ opératoire strictement nécessaire, ceci étant du à une intensité de faisceau faible, à une collimation précise et à une utilisation d'un détecteur de rayons X sensible, disposé très près de la zone à analyser, et pourvu d'un blindage arrière.

Les photons qui sont émis par le scintillateur sont acheminés vers l'amplificateur de luminance, qui donne un gain de luminance important à l'image produite. Celle-ci est reprise par un dispositif à transfert de charges qui transforme les informations optiques de l'image en signaux électriques d'image traités par un dispositif de traitement électronique, pour être visualisés sur l'écran d'un téléviseur et/ou stockés dans un dispositif de stockage, par exemple la bande magnétique d'un magnétoscope.

Un dispositif à transfert de charges bidimensionnel est un composant électronique de très faibles dimensions qui permet de stocker, en bloc, toutes les informations d'une image, celles-ci étant restituées par la suite en effectuant une lecture séquentielle de chaque ligne de stockage de charges du dispositif à transfert de charges. Sur le plan de l'encombrement et du poids, le dispositif à transfert de charges n'intervient quasiment pas au niveau de l'outil dentaire, ceux-ci se réduisent alors à ceux de l'amplificateur de luminance et du conduit d'images à fibres optiques.

Il est ainsi possible d'acquérir une trame d'image de télévision en un temps de 20 millisecondes avec une source de rayons X travaillant avec un courant de 0,2 mA sous 65 kV alors que les techniques photographiques nécessitent des temps de 0,2 à 1 seconde environ pour une source de rayons X travaillant avec un courant de 6 mA sous 65 kV.

A temps de pose équivalent, la dose de rayons X nécessaire à l'acquisition d'une image unique se trouve ainsi réduite d'un facteur 30 environ.

En outre, si l'on considère le temps d'acquisition de 20 millisecondes comparé aux temps de 0,2 à 1 seconde pratiqués dans les systèmes photographiques conventionnels, nécessaires pour obtenir une qualité d'image comparable la durée du temps d'exposition se trouve ainsi réduite d'un facteur 10 à 50. Par conséquent, globalement, les diminutions conjointes du courant de source et de la durée d'acquisition permettent d'atténuer la dose de rayons X d'un facteur 300 à 1500.

Le conduit d'images à fibres optiques doit assurer une transmission avec une définition d'image suffisante. Des conditions correctes d'observation ont été obtenues avec un conduit d'images ayant des fibres optiques espacées avec un pas d'environ 6 µm, avec lequel il est possible de distinguer sur l'image finale des objets métalliques, tels que des fils, ayant un diamètre de 60 µm.

L'observation en temps réel sur l'écran du téléviseur permet au praticien d'opérer la prise des images utiles après un réglage préalable aisés, car il lui suffit de placer l'outil dentaire en observant sa position sur l'écran du téléviseur. Il peut opérer par des prises successives et courtes d'images uniques qu'il restitue à l'aide du magnétoscope. Il peut ainsi positionner parfaitement le conduit d'images à fibres optiques, ce qui permet de collimater la source de rayons X sur la seule zone à étudier alors que les techniques habituelles nécessitent un faisceau couvrant une plus grande surface lorsque l'on ne dispose pas de cette possibilité de réglage en temps réel ou de préréglage dans la disposition en arceau.

Le flux de rayons X et donc la dose est très faible dans la zone à étudier, et la dose de rayons X diffusée est abaissée de sorte que le praticien se trouve ainsi protégé.

L'amplificateur de luminance à focalisation de proximité est de type classique constitué d'une photocathode d'entrée effectuant la transformation photons-électrons, puis d'un multiplicateur d'électrons par exemple à microcanaux, et d'un écran luminescent. Cet écran est couplé au dispositif à transfert de charges qui effectue la transformation de l'image de l'écran luminescent en des signaux électriques d'images visualisés sur le téléviseur à l'aide d'un dispositif de traitement électronique.

L'invention sera mieux comprise à l'aide des figures suivantes qui représentent :

Figure 1 : une vue en coupe d'un conduit d'images rigide à fibres optiques droit avec l'extrémité en bouche portant le scintillateur sur une face biseautée.

Figure 2 : une vue en coupe d'un conduit d'images rigide è fibres optiques muni dans la zone en bouche d'un raccord de conduit d'images à fibres optiques à renvoi d'angle portant à son extrémité le scintillateur.

Figure 3 : une vue de l'extrémité du conduit d'images rigide à fibres optiques portant le scintillateur muni d'un raccord de conduit d'images à fibres optiques à concentration et à renvoi d'angle.

Figure 4 : une vue de l'outil dentaire comprenant le conduit d'images rigide à fibres optiques relié par un raccord de conduit d'images à fibres optiques à concentration à un conduit d'images souple à fibres optiques réuni à l'amplificateur de luminance puis au dispositif à transfert de charges.

Figure 5 : une vue de l'outil dentaire comprenant un conduit d'images rigide à fibres optiques associé à un amplificateur d'images rigide à fibres optiques associé à un amplificateur de luminance et à un dispositif à transfert de charges.

Figure 6 : une vue de l'outil dentaire mis en bouches positionnés par un arceau, par rapport à la source de rayons X.

Figure 7 : une représentation de la chaîne d'acquisition d'images comprenant un outil dentaire, un dispositif de traitement électronique un téléviseur et un magnétoscope.

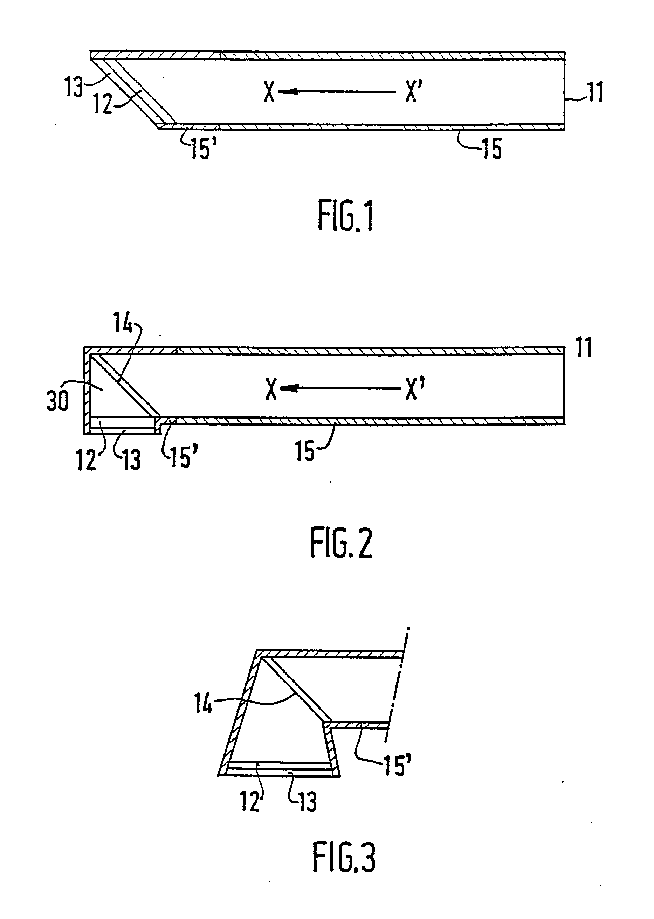

Sur la figure 1 est représenté un conduit d'images rigide à fibres optiques 11 dont l'extrémité destinée à être mise en bouche est munie d'un scintillateur 12 ayant un support protecteur 13. L'ensemble est protégé par une gaine 15, 15' destinée à assurer la protection mécanique du conduit d'images rigide à fibres optiques 11 et à empêcher à tout rayonnement photonique parasite de pénétrer dans le conduit d'images rigide à fibres optiques 11, et pour la partie 15' de la gaine, à absorber, en outre, les rayonnements X de la source de rayons X qui n'auraient pas été convertis par le scintillateur. La gaine 15, 15' et le support protecteur 13 qui lui est fixé, par exemple par une colle époxyde, servent aussi de protection contre les agents corrodants lors des opérations de stérilisation du conduit d'images rigide à fibres optiques 11. Celui-ci est biseauté pour faciliter sa manipulation dans la cavité buccale.

La face biseautée fait un certain angle avec la direction X X' représentant la direction de tenue en bouche. Cet angle peut varier dans de grandes proportions c'est-à-dire que des outils différents peuvent utiliser des angles différents en fonction de la zone à explorer à laquelle ils sont destinés. La restitution des proportions de l'objet implique l'application d'une règle d'isométrie. Ceci revient à dire que la bissectrice de l'angle formé par la direction X X' et la direction du faisceau de rayons X incident doit passer sensiblement par le plan du scintillateur 12. Si la direction du faisceau de rayons X incident coïncide avec la direction X' la valeur de l'angle du biseau n'intervient plus.

Sur la figure 2 est représenté le conduit d'images rigide à fibres optiques 11 muni dans la zone en bouche d'un raccord d'images à fibres optiques 30 constitué d'un renvoi d'angle. Celui-ci porte le scintillateur sur une face sensiblement parallèle à la direction X X' représentant la direction de tenue en bouche. Cette disposition présente l'intérêt d'assurer un faible encombrement à la partie en bouche de l'outil dentaires et autorise la prise d'images photoniques sous des angles variés nécessités par l'application elle-même. Des moyens optiques d'adaptation d'indices 14 sont disposés entre le conduit d'images rigide 11 et le raccord de conduit d'images 30.

Sur la figure 3 est représentée selon une variante l'extrémité qui pénètre en bouche du conduit d'images rigide à fibres optiques 11. Cette extrémité est munie d'un raccord de conduit d'images à fibres optiques à concentration 30 qui supporte le scintillateur 12. Ce raccord de conduit d'images à fibres optiques est relié au conduit d'images rigide à fibres optiques 11 par un moyen optique d'adaptation d'indices 14 constitué, par exemple, d'une mince pellicule d'huile silicone ou de baume du Canada, ou de produits donnant le même résultat. La gaine 15, 15' assure également dans ce cas la fonction de tenue mécanique de l'ensemble.

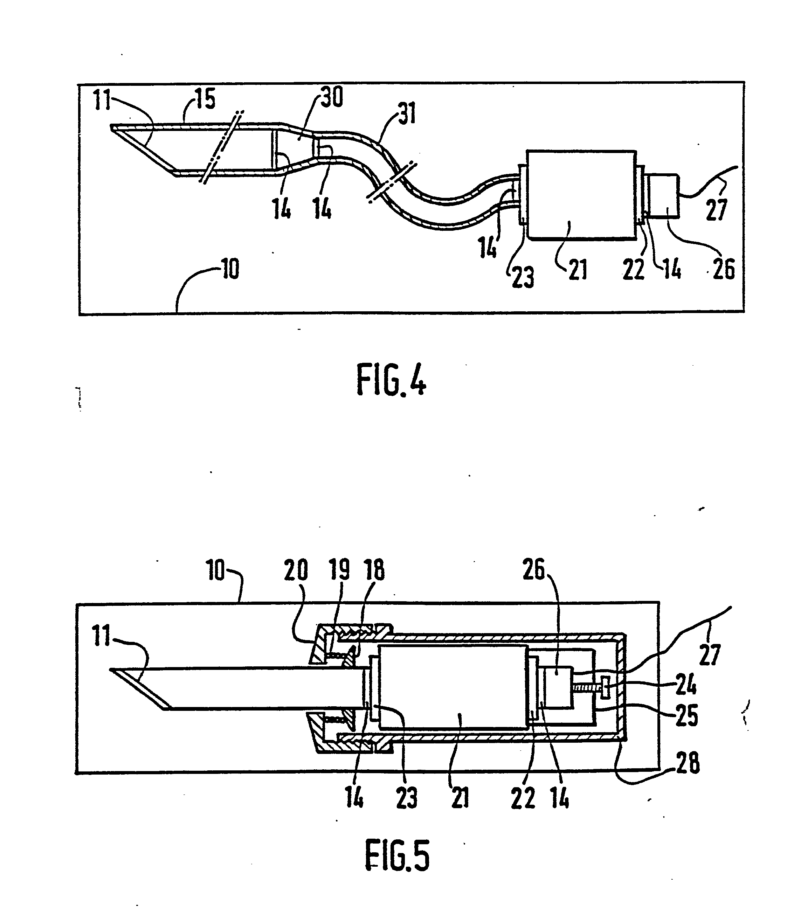

Sur la figure 4 est représenté l'outil dentaire 10 comprenant le conduit d'images rigide à fibres optiques 11 relié par un raccord de conduit d'images à fibres optiques 30 muni de moyens optiques d'adaptation d'indices 14, à un conduit d'images souple à fibres optiques 31 qui est réuni à l'amplificateur de luminace 21 puis au dispositif à transfert de charges 26. L'amplificateur de luminance 21 possède une fenêtre d'entrée 23 et une fenêtre de sortie 22. Pour réunir optiquement ces fenêtres respectivement au conduit d'images souple à fibres optiques 31 et au dispositif à transfert de charges 26 il est nécessaire d'interposer également des moyens optiques d'adaptation d'indices 14 constitués par exemple d'une lame d'huile silicone ou de baume du Canada ou de produits donnant le même résultat. Le conduit d'images souples à fibres optiques 31 possède une longueur suffisante par exemple environ 1 mètre, pour permettre au praticien de disposer de suffisamment de liberté de mouvement pour opérer. l'amplificateur de luminance 21 et le dispositif à transfert de charges 26 se trouvent alors situés dans la partie de l'appareillage dentaire disposée à proximité. Un faisceau électrique 27 regroupe les signaux électriques d'alimentation de contrôle et d'informations d'images.

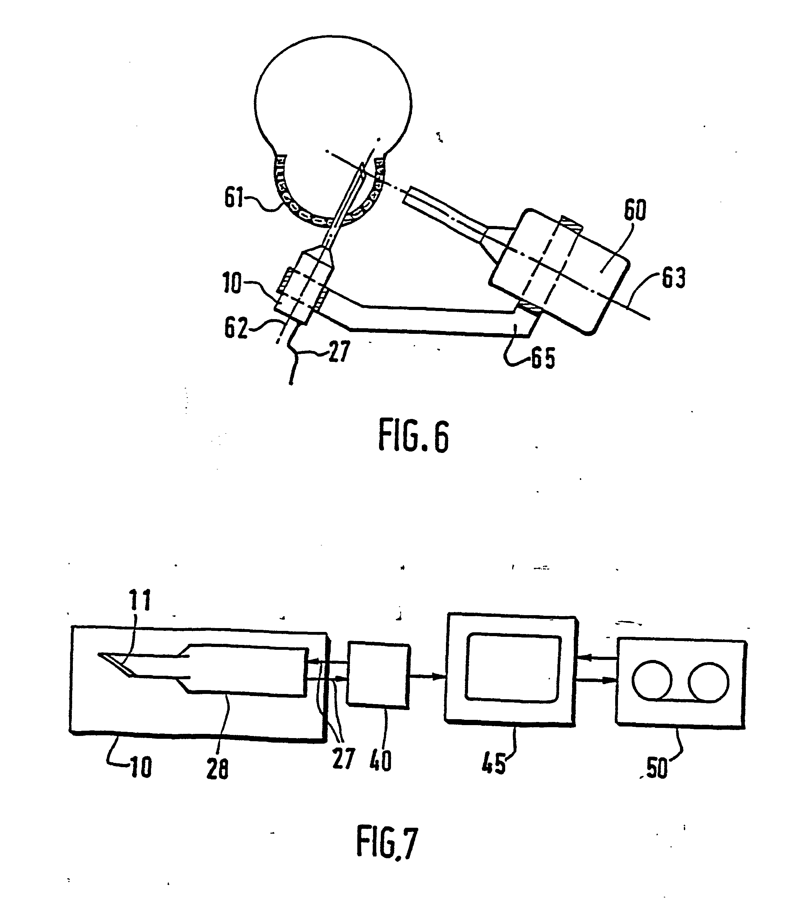

Sur la figure 5 est représenté l'outil dentaire 10 selon une variante compacte. Il comprend le conduit d'images rigide à fibres optiques 11 muni du scintillateur 12, du support protecteur 13, et de la gaine 15, 15' (voir figure 1), le conduit d'images rigide à fibres optiques pouvant être fixé mécaniquement à un boîtier 28 à l'aide d'une bague filetée 20. Celle-ci vient s'appuyer, par l'intermédiaire du joint élastique 19 sur une portée hémisphérique 18 solidaire du conduit d'images rigide à fibres optiques 11, tout en étant vissée au boîtier 28. A l'intérieur de celui-ci, se trouve monté l'amplificateur de luminance 21 de telle sorte que lorsque le montage est achevé l'extrémité du conduit d'images rigide à fibres optiques 11 qui fait face à l'amplificateur de luminance 21 se trouve être mise optiquement en contact avec la fenêtre d'entrée 23 de l'amplificateur de luminance par des moyens optiques d'adaptation d'indices 14 tels que par exemple une mince lame d'huile silicone ou de baume du Canada ou autres. Le dispositif à transfert de charges 26 est fixé mécaniquement à l'aide d'une vis de poussée 24 montée sur un cavalier 25 solidaire de l'enveloppe de l'amplificateur de luminance 21. A travers le boîtier 28 passe un faisceau électrique 27 regroupant les signaux électriques d'alimentations de contrôle et d'informations d'images.

Sur la figure 4 est représenté un raccord de conduit d'images à fibres optiques à concentration 30 destiné à adapter les dimensions des faces de sortie des conduits d'images rigide et souple à fibres optiques 11, 31, afin de ne pas perdre d'informations d'images. Dans cette fonction ce raccord de conduit d'images est réuni à chaque extrémité par un moyen optique d'adaptation d'indices 14, par exemple une lame d'huile silicone ou de baume du Canada ou autres. Un tel raccord de conduit d'images en assurant la même fonction peut également être utilisé à d'autres jonctions optiques de l'outil dentaire sans s'écarter du but de l'invention. Ainsi la jonction optique entre le conduit d'images souple à fibres optiques 31 et la fenêtre d'entrée 23 de l'amplificateur de luminance 21 ou encore la jonction optique entre la fenêtre de sortie 22 de l'amplificateur de luminance 21 et le dispositif à transfert de charges 26, peuvent utiliser également un raccord de conduit d'images à fibres optiques muni des mêmes moyens optiques d'adaptation d'indices.

Il est d'autre part évident que sans sortir du but de l'invention le raccord de conduit d'images à fibres optiques 30 présenté sur la figure 4 sous une forme à concentration peut également être remplacé par l'homme de l'art, dans tous les cas cités en fonction des dimensions des surfaces à raccorder optiquement, par un raccord de conduit d'images à épanouissement ou par un raccord de conduit d'images à simple transfert d'images.

Sur la figure 6 est représentée une vue de l'outil dentaire la monté sur un arceau 65 fixé à la source de rayons X 60. Cette fixation rigide de la source de rayons X 60 et de l'outil dentaire est telle que les axes de symétrie respectifs 63, 62 de ces derniers concourent juste en arrière du scintillateur. L'angle que font ces deux axes entre eux est voisin de 90°. La figure 6 représente une mâchoire inférieure 61 vue de dessus avec l'outil dentaire disposé sur la partie gauche de cette mâchoire inférieure. L'outil dentaire 10, en étant fixé à la source de rayons X 60 par l'intermédiaire de l'arceau 65, utilise la mobilité assurée par la potence qui soutient la source pour pouvoir être déplacé par le praticien dans tout le champ opératoire. Le positionnement de l'outil dentaire par rapport à la source de rayons X est ainsi préréglé de sorte que les réglages d'intensité de faisceau, et de collimation peuvent être prédéterminés pour la disposition adoptée.

La figure 7 représente la chaîne d'acquisition d'images comprenant un outil dentaire 10, un dispositif de traitement électronique 40, un téléviseur 45 et un magnétoscope 50.

L'outil dentaire 10 échange, avec le dispositif de traitement électronique 40 par l'intermédiaire du faisceau électrique 27, des signaux constitués des signaux d'alimentation et de contrôle que reçoit l'outil dentaire et des signaux d'information d'images que restitue l'outil dentaire. Ces derniers sont traités par le dispositif de traitement électronique 40 afin de les transformer en signaux vidéofréquences compatibles avec les caractéristiques techniques du téléviseur 45 ou d'un moniteur et du magnétoscope. Ce dernier peut être utilisé pour enregistrer des images par l'intermédiaire ou non du téléviseur et simultanément ou non avec le téléviseur. Il peut être utilisé pour restituer ultérieurement après les prises de vues, les images qu'il permet de stocker. Le magnétoscope peut être remplacé par tout autre dispositif de stockage tel qu'un disque numérique une mémoire électronique etc...

## Revendications

1. Appareillage dentaire de détection de rayons X permettant d'acquérir en temps réel des images d un champ opératoire couvert par un faisceau de rayons X issu d'une source de rayons X (60), comprenant un scintillateur (12), placé sur une extrémité d'un conduit d'image à fibres optiques (11) dont l'autre extrémité est reliée à un amplificateur de luminance (21) couplé à un dispositif de prise de vue électronique (26), le scintillateur (12) étant placé en bouche, caractérisé en ce que la partie en bouche du conduit d'image à fibres optiques (11) présente une face biseautée formée par une section non-droite du conduit d'images de sorte que la direction de tenue en bouche du conduit d'image (11) n'est pas colinéaire avec la direction du faisceau de rayons X, et en ce que le dispositif de prise de vue (28) est un dispositif à transfert de charges bidimensionnel.

2. Appareillage dentaire selon la revendication 1, caractérisé en ce que le scintillateur (12) est déposé sur la face biseautée du conduit d'image à fibres optiques (11).

3. Appareillage dentaire selon la revendication 1, caractérisé en ce que la face biseautée est accolée à une face d'un élément à renvoi d'angle à fibres optiques (30) dont l'autre face porte le scintillateur (12).

4. Appareillage dentaire selon la revendication 3, caractérisé en ce que l'élément à renvoi d'angle est à concentration.

5. Appareillage dentaire selon une des revendications 1 à 5, caractérisé en ce que le conduit d'image est constitué d'une partie rigide (11) qui porte le scintillateur (12) et d'une partie souple (31) reliée à l'amplificateur de luminance (21).

6. Appareillage dentaire selon l'une des revendications 4 à 5, caractérisé en ce que la partie rigide (11) et la partie souple (31) du conduit d'image sont réunies par un raccord de conduit d'images à fibres optiques (30) à épanouissement ou à concentration ou à transfert direct ou à renvoi d'angle.

7. Appareillage dentaire selon une des revendications 1 à 6, caractérisé en ce que le conduit d'images à fibres optiques (11), l'amplificateur de luminance (21), le dispositif à transfert de charges (26) et le scintillateur (12) font partie d'un outil dentaire.

8. Appareillage dentaire selon la revendication 7, caractérisé en ce que la source de rayons X (60) et l'outil dentaire (10) sont positionnés rigidement l'un par rapport à l'autre à l'aide d'un arceau (65) afin de pouvoir les déplacer solidairement de sorte que leurs axes respectifs de symétrie concourent juste en arrière du scintillateur (12).

9. Appareillage dentaire selon la revendication 8 dans la mesure où elle dépend de la revendication 2, caractérisé en ce que la bissectrice de l'angle formé par les deux axes est sensiblement située dans le plan du scintillateur.

10. Appareillage dentaire selon les revendications 8 ou 9, caractérisé en ce que l'angle des axes respectifs est voisin de 90°.

**Claims**

1. A dental X-ray detection apparatus enabling real-time acquisition of images from an operating field covered by an X-ray beam emitted by an X-ray source (60), comprising a scintillator (12) which is arranged at one end of a fibre-optic image conductor (11) whose other end is connected to a light amplifier (21) which is coupled to an electronic image pick-up device (26), the scintillator (12) being arranged inside the mouth, characterized in that the mouth portion of the fibre-optic image conductor (11) comprises a bevelled surface which is formed by a non-straight section of the image conductor so that the direction of holding of the image conductor (11) in the mouth is not colinear with the direction of the X-ray beam, the image pick-up device (26) being a two-dimensional charge transfer device.

2. A dental apparatus as claimed in claim 1, characterized in that the scintillator (12) is arranged on the bevelled surface of the fibre-optical image conductor (11).

3. A dental apparatus as claimed in Claim 1, characterized in that the bevelled surface is joined to one face of a fibre-optic angular reflection element (30), the other face of which carries the scintillator (12).

4. A dental apparatus as claimed in claim 3, characterized in that the angular reflection element is a concentration-type element.

5. A dental apparatus as claimed in any one of the Claims 1 to 5, characterized in that the image conductor is formed by a rigid part (11) which carries the scintillator (12) and a flexible part (31) which is connected to the light amplifier (21).

6. A dental apparatus as claimed in one of the Claims 4 or 5, characterized in that the rigid part (11) and the flexible part (31) of the image conductor are interconnected by means of a fibre-optic image conductor connector (30) of the dispersion type, the concentration type, the direct transfer type or the angular reflection type.

7. A dental apparatus as claimed in any one of the claims 1 to 6, characterized in that the fibre-optic image conductor (11), the light amplifier (21), the charge transfer device (26) and the scintillator (12) form part of a dental tool.

8. A dental apparatus as claimed in Claim 7, characterized in that the X-ray source (60) and the dental tool (10) are rigidly mounted with respect to one another by means of a bow (65) in order to be displaceable together so that their respective symmetry axes converge just behind the scintillator (12).

9. A dental apparatus as claimed in Claim 8 in as far as it depends on Claim 2, characterized in that the bisector of the angle enclosed by the two axes situated substantially in the plane of the oscillator.

10. A dental apparatus as claimed in Claim 8 or 9, characterized in that the angle enclosed by the respective axes is approximately 90°.

**Patentansprüche**

1. Zahnärztliche Einrichtung zur Dektion von Röntgenstrahlung, mit der Bilder eines von einem Röntgenstrahl aus einer Röntgenquelle (60) abgetasteten Funktionsgebiets in Echtzeit gewonnen werden können, mit einem Szintillator (12) auf einem Ende eines aus Lichtleitfasern bestehenden Bildkanals (11), dessen anderes Ende an einen Leuchtdichteverstärker (21) angeschlossen ist, der selbst mit einem elektronischen Bildaufnehmers (26) verbunden ist, wobei der Szintillator (12) in den Mund eingeführt wird, dadurch gekennzeichnet, daß der im Mund befindliche Teil des Lichtleitfaser-Bildkanals (11) eine durch einen ungeraden Abschnitt des Bildkanals gebildete Schrägfläche derart darstellt, daß die Aufstellungsrichtung des Bildkanals (11) im Mund nicht linear zur Richtung des Röntgenstrahls verläuft, und daß der Aufnehmer (26) eine Einrichtung zum zweidimensionalen übertragen von Ladungen ist.

2. Zahnärtzliche Einrichtung nach Anspruch 1, dadurch gekennzeichnet, daß der Szintillator (12) auf der Schrägfläche des Lichtleitfaser-Bildkanals (11) angeordnet ist.

3. Zahnärtzliche Einrichtung nach Anspruch 1, dadurch gekennzeichnet, daß die Schrägfläche an einer Fläche eines lichtleitfaser-Winkelablenkelements (30) angebaut ist, dessen andere Fläche den Szintillator (12) trägt.

4. Zahnärtzliche Einrichtung nach Anspruch 3, dadurch gekennzeichnet, daß das Winkelablenkelement ein Bündelungselement ist.

5. Zahnärtzliche Einrichtung nach einem der Ansprüche 1 bis 5, dadurch gekennzeichnet, daß der Bildkanal besteht aus einem starren Teil (11), der den Szintillator (12) trägt, und aus einem elastischen Teil (31), der mit dem Leuchtdichteverstärker (21) verbunden ist.

6. Zahnärtzliche Einrichtung nach einem der Ansprüche 4 und 5, dadurch und 5, dadurch gekennzeichnet, daß der starre Teil (11) und der elastische Teil (31) des Bildkanals durch ein Übergangsstück (30) am Licht-leitfaser-Bildkanal im Streuungsbetrieb, im Bündelungsbetrieb, für Direktübertragung oder mit Winkelablenkung miteinander verbunden sind.

7. Zahnärtzliche Einrichtung nach einem der Ansprüche 1 bis 6, dadurch gekennzeichnet, daß der Bildkanal (11), der Leuchtdichteverstärker (21), die Ladungsübertragungseinrichtung (26) und der Szintillator (12) Bauteile eines zahnärtzlichen Geräts sind.

8. Zahnärtzliche Einrichtung nach Anspruch 7, dadurch gekennzeichnet, daß die Röntgenquelle (60) und das zahnärtzliche Gerät (10) über einen Bügel (65) starr miteinander verbunden sind, um sie gleichzeitig so bewegen zu können, daß ihre Symmetrieachsen genau hinter dem Szintillator (12) zur Deckung kommen.

9. Zahnärtzliche Einrichtung nach Anspruch 8, sofern abhängig vom Anspruch 2, dadurch gekennzeichnet, daß die Halbierende des von den beiden Achsen gebildeten Winkels sich im wesentlichen in der Ebene des Szintillators befindet.

10. Zahnärtzliche Einrichtung nach Anspruch 8 oder 9, dadurch gekennzeichnet, daß der Winkel der betreffenden Achsen ungefähr 90° beträgt.

FIG.1

FIG.2

FIG.3

1

FIG.4

FIG.5

2

FIG.6

FIG,7